# EUROPEAN PATENT SPECIFICATION

(11) **EP 4 103 112 B1**
(45) Date of publication and mention of the grant of the patent: **11.11.2026**
(21) Application number: 20918647.7
(22) Date of filing: 07.10.2020
(51) Int. Cl.: A61F 2/90, A61F 2/01, A61B 17/221, A61F 2/95

(54) **TEMPORARY AND RETRIEVABLE EXPANDABLE MEMBER**
VORÜBERGEHENDES UND RÜCKHOLBARES EXPANDIERBARES ELEMENT
ÉLÉMENT EXPANSIBLE TEMPORAIRE ET RÉCUPÉRABLE

(30) Priority: 11.02.2020 US 202016787573
(43) Date of publication of application: 21.12.2022
(73) Proprietor: Amaitus, Inc., Palo Alto, CA 94306 (US)
(72) Inventor: NIKANOROV, Alexander, Palo Alto, CA 94306 (US); ZHAO, Hugh, Qinghong, Pleasanton, CA 94588 (US)
(74) Representative: Mathys & Squire
(86) International application number: PCT/US2020/054546
(87) International publication number: WO 2021/162756

(56) References cited:
- US-A- 5 222 971
- US-A- 5 972 019
- US-A1- 2004 122 466
- US-A1- 2004 122 466
- US-A1- 2005 149 159
- US-A1- 2007 225 789
- US-A1- 2009 264 988
- US-A1- 2011 224 707
- US-A1- 2014 343 585
- US-A1- 2015 272 716
- US-A1- 2016 256 304
- US-A1- 2019 254 846
- US-A1- 2019 254 846

## Description

### CLAIM OF PRIORITY

The present application claims the benefit of US Patent Application No. 16/787,573 (Attorney Docket No. 5392.001US1) filed on February 11, 2020.

### BACKGROUND

Narrowing or occlusion of body lumens or other passageways are a common medical condition, specifically in the blood vessels (e.g. arteries and veins) and airways (e.g. nasal passageway, sinuses, trachea, bronchi, etc.). Other examples include but are not limited to peripheral artery disease (PAD) including the superficial femoral artery (SFA), popliteal artery, vessels above or below the knee (A/K or BTK), iliac artery, aorta, renal arteries, coronary artery disease (CAD), and heart valves, etc. Treatment options may include one or more of bare metal stents, drug eluting stents, absorbable stents, plain old balloon angioplasty (POBA), drug coated balloons (DCB), open surgery, and other treatments known in the art.

Coronary artery disease (CAD) also referred to as coronary heart disease (CHD) is the most common type of heart disease, killing over 370,000 people annually. Every year about 735,000 Americans have a heart attack. Of these, 525,000 are a first heart attack and 210,000 happen in people who have already had a heart attack. Current treatments include but are not limited to stenting, POBA, etc.

In the case of PAD, an estimated 200 million patients worldwide suffer from this condition, including 20 million patients in the USA. PAD has a five-year mortality rate of about 30% and is estimated to cost the US healthcare system approximately $319 billion annually. The average cost to treat a Medicare beneficiary was over $11,000 per patient in 2015 as compared to the average PAD patient cost of over $72,000.

Obstruction or blockages of lumens or other body channels is not limited to the vascular system and may include other lumen occlusions and obstructions such as the trachea, nasal passageways, or any other lumen or body channel where fluids such as a gas or liquid pass through.

US 2015/272716 describes an endovascular microvalve device for use in a vessel during a therapy procedure. The device includes an outer catheter, an inner catheter displaceable within the outer catheter, and a filter valve coupled to the distal ends of the inner and outer catheters. The valve is constructed of a braid of elongate first filaments coupled together at their proximal ends in a manner that the first filaments are movable relative to each other along their lengths. A filter is provided to the braid formed by electrostatically depositing or spinning polymeric second filaments onto the braided first filaments.

US 2019/254846 describes a catheter system and methods of using such catheter system for the treatment of vascular and non-vascular pathologies, more specifically a catheter system for the actuation of a temporarily implantable member elastically deformable by hydraulic means.

US 2004/122466 describes a filter basket device which includes two shaft wires, a filter basket adapted to capture emboli and thrombi, and an actuation handle. The shaft wires are loosely twisted together so that they remain adjacent to each other, and yet are able to slide relative to each other with little friction and without plastic deformation. One shaft wire is connected to the distal end of the filter basket, and the other shaft wire is connected to the proximal end of the filter basket. The actuation handle moves the shaft wires relative to each other to control opening and closing of the basket. In one embodiment, a guidewire-accepting fitting is attached to a distal portion of the device and permits advancement of the device over a guidewire until a desired location along the guidewire is reached.

US 2011/224707 describes a device for opening occluded blood vessels, comprising a braided structure on a guide wire, said braided structure being securely and permanently attached to the guide wire and consisting of a plurality of filaments arranged in a helical manner and at least at the proximal end combined into a bundle, with the braided structure assuming an elongated form of smaller diameter when subjected to external force while when in unstressed state defining a tubular element of larger diameter closed at least at its proximal end, with the device being provided with restraining/bracing fiber elements.

The present invention is set out in the appended claims.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Fig. 1 illustrates an example of a radially expandable member.
Fig. 2 illustrates a proximal portion of the radially expandable member in Fig. 1.
Figs. 3A and 3B illustrate the radially expandable member of Fig. 1 in the collapsed and expanded configurations, respectively.
Fig. 4A shows a side view of an asymmetrical radially expandable member.
Fig. 4B shows a perspective view of the radially expandable member in Fig. 4A.
Fig. 4C shows and end view of the radially expandable member in Fig. 4A.
Figs. 4D-4G show additional examples of a radially expandable member.
Fig. 4H shows an example of a radially expandable member in a body cavity.
Fig. 5A shows side view of an example of the helical filaments in a radially expandable member in the expanded configuration.
Fig. 5B shows a side view the radially expandable member of Fig. 5A in the collapsed configuration.
Fig. 5C shows a side view of a radially expandable member with an additional helical filament added to the example of Fig. 5A.
Fig. 5D shows a side view of a radially expandable member with an additional helical filament added to the example of Fig. 5C.
Figs. 6A-6C illustrate examples of overlapping filaments.
Fig. 7A illustrates an example of a resilient joint formed between filaments.
Fig. 7B shows woven filaments forming an expandable tubular body.
Fig. 7C shows integrally connected struts forming an expandable tubular body.
Fig 8A illustrates a side view of compression of an expandable tubular body due to inadequate radial strength.
Fig. 8B illustrates a side view of uniform radial strength of an expandable tubular body.
Figs. 9A-9D illustrate side views of various examples of expanded radial tubular bodies.
Fig. 10 illustrates a side view of an example of an expanded radial tubular body with varying diameter.
Fig. 11A illustrates a side view of an example of a handle for actuating the radially expandable member.
Fig. 11B illustrates a side view of an example of an actuator in a handle.
Fig. 11C illustrates an example of a handle with an actuator coupled to an expandable member.
Fig. 11D highlights a distal region of the handle in Fig. 11C.
Figs. 12A-12I illustrate side views of various actuator configurations on a handle.
Fig. 13 shows an example of a handle.
Fig. 14 shows another example of a handle.
Figs. 15A-15E illustrate enlargement of proximal and/or distal openings in an expandable member.

### DETAILED DESCRIPTION

Narrowing or occlusion of body lumens are a common medical condition, specifically in the blood vessels (e.g. arteries and veins) and airways (e.g. nasal passageway, sinuses, trachea, bronchi, etc.). Other examples include but are not limited to Peripheral artery disease (PAD) including the superficial femoral artery (SFA), popliteal artery, vessels above or below the knee (A/K or BTK), iliac artery, aorta, renal arteries, coronary artery disease (CAD), heart valves, etc. Treatment options may include one or more of bare metal stents, drug eluting stents, absorbable stents, plain old balloon angioplasty (POBA), drug coated balloons (DCB), open surgery, and other treatments known in the art.

Obstruction or blockages of body channels is not limited to the vascular system and may include other lumen occlusions and obstructions including the trachea, nasal passageways, esophagus, gastrointestinal tract, or any other lumen or body channel where fluids such as a gas or a liquid pass through.

Each of these treatments have their advantages and disadvantages. It would be desirable to provide improved devices, methods, and systems for treating at least some of these diseases.

The examples disclosed herein generally relate to the field of medical devices, systems, and methods. More particularly, the devices, systems and methods are described in relation to intraluminal devices such as endovascular, intraarterial, intravenous, endolymphatic, endotracheal, endobronchial, nasal, sinusoidal, ductal, ureteral) support scaffold-like devices for temporary placement and removal. Other applicable devices, systems and methods may be related to lumen wall support and medicinal delivery that can perform the functions of repeated expanding and collapsing, and so that the device can be delivered upon radially collapsing into a lower profile that can be delivered to a targeted anatomy and/or lesions, then radially expanded to support the lumen opening for a desirable period of time, repositioned upon radially collapsing and re-expanding, and then retrieved upon re-collapsing, and its preparation method and use thereof. These examples are of course not intended to be limiting and one of skill in the art will appreciate that the devices, systems, and methods disclosed herein may be used in many other applications.

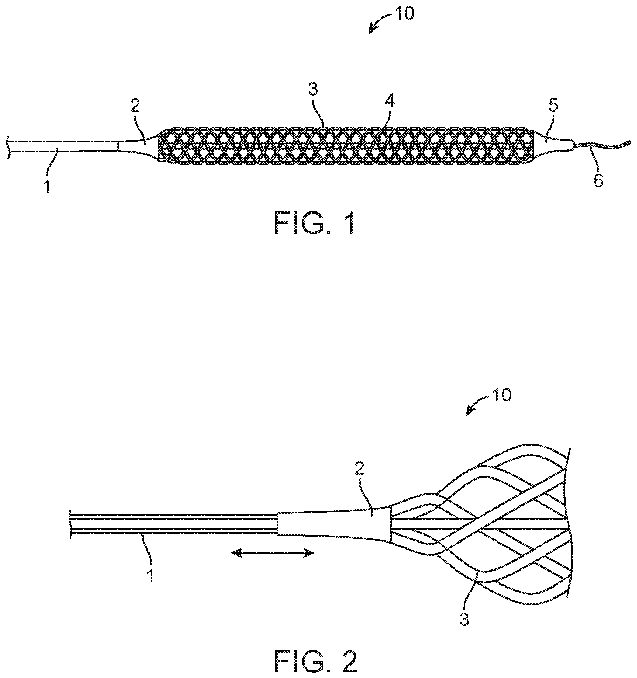

Fig. 1 shows an example of a scaffold-like device 10 formed from a plurality of superimposed helical filaments 3 such as wires. Fig. 1 shows the device 10 in the expanded state and includes an outer shaft 1, a proximal portion of the device 2 with the outer shaft 1 (also referred to as outer tube) coupled to the proximal portion of helical filaments 3. Inner shaft 4 is disposed under the expandable tubular body formed by the helical filaments and may be coaxially disposed under the expandable tubular body. The distal portion of the helical filaments is coupled to a distal portion of the inner shaft to form a distal joint 5. An optional guidewire 6 may be disposed through a lumen in the inner shaft 4, and the inner shaft may extend slidably through a lumen in the outer shaft 1. The helical filaments form a hollow tubular body with apertures in the sidewall which allow fluids such as a gas like air, or a fluid like blood to flow through the device when it is collapsed and being delivered, or when it is expanded and engaged with the walls of the lumen or other treatment site. An outer layer of heat shrink tubing may be applied at the proximal and distal ends of the tubular body where the filaments are coupled to the inner and outer shafts to provide a smoother, atraumatic surface at the proximal and distal ends.

The scaffold-like design uses superimposed helical wires having distal ends joined with the tip of an inner shaft (also referred to herein as an inner member) such as a hypotube, and the proximal ends of the helical wires are joined to an outer shaft (also referred to herein as an outer member) such as sheath. The superimposed helical scaffold-like device may be fabricated from any type of filament such as thin wires or threads with any diameter, including a diameter ranging from 50 to 500 microns, although any size may be used. The outer shaft may be made of a thin-walled tube with good resistance to kinking. The inner shaft may be made of a hypotube or any other material that can be slidably passed through the lumen of outer member. The diameter of the inner tube may range from 150 to 1500 microns, although this is not intended to be limiting. Relative movement of the inner shaft and outer shaft will actuate the plurality of filaments to either expand or collapse the tubular body formed by the filaments, as will be discussed in greater detail below.

Fig. 2 illustrates a close-up of the proximal portion of the expandable device 10 illustrated in Fig. 1 above where the joint is formed using techniques known in the art such as adhesive bonding, welding, or other methods. The proximal portion 2 of superimposed helical wires 3 are fixed onto the end portion of the outer shaft 1, and an inner member slides inside a lumen of the outer shaft via a distal pushing or a proximal pulling motion as indicated by the arrow, passing under the helical filaments. At the proximal joint, the helical wires are welded, bonded, or otherwise fixedly coupled to the outside surface of the outer member which may have a wall thickness ranging from 100 to 500 microns, although this is not intended to be limiting. The number of superimposed helical wires may range from 2 to 12 depending on the helix pattern, although this is not intended to limit the number of filaments used. An outer heat shrink tubing or other sleeve may be disposed over the proximal end of the plurality of filaments to provide a smooth transition from the outer shaft to the filament region. The distal joint may be similarly formed or may be formed using other techniques known in the art.

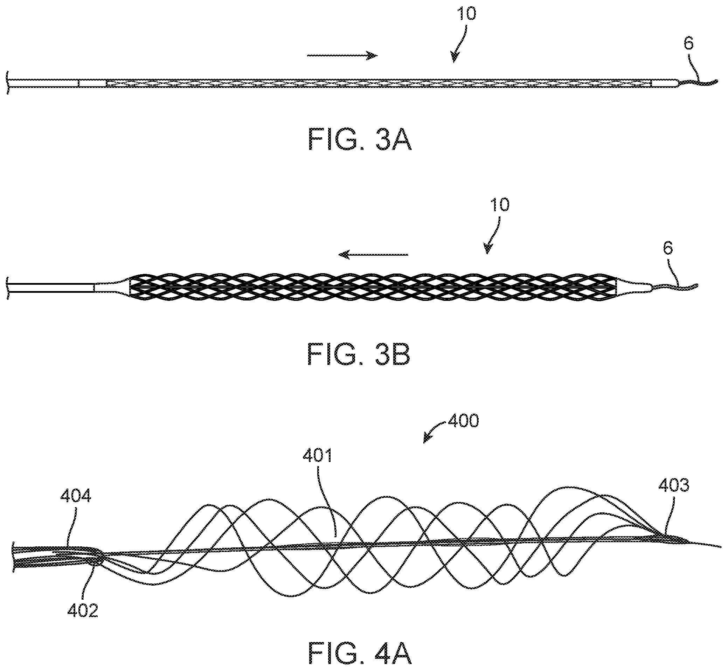

Figs. 3A and 3B illustrate the expandable member 10 of Fig. 1 in the collapsed and expanded configurations, respectively. Optional guidewire 6 is also show extending from the inner shaft. The superimposed helical scaffold-like device may be radially expanded by moving the inner shaft relative to the outer shaft, for example by pulling the inner movable shaft proximally into the outer stable shaft as illustrated by the arrow in Fig. 3B. This applies compression to the plurality of helical filaments which then bow radially outward to radially expand and form the tubular body. Continued movement of the inner shaft relative to the outer shaft will further increase the diameter of the tubular body until a maximum diameter is obtained. Additionally, with increased diameter, the tubular body will exert higher radial force as it engages and supports tissue. Since relative motion is required to actuate the expandable member, a similar result may be obtained by pushing the outer shaft over the inner shaft to compress and expand the filaments, or a combination of pushing and pulling the inner and outer shafts relative to one another may also be used to expand the filaments into the tubular body.

Actuation of the inner and outer shafts relative to one another in the opposite direction as required to expand the device will also radially collapse the expanded tubular body and reduce the applied radial force when the compression on the helical filaments is released and tension is applied to the helical filaments. For example, the inner shaft may be advanced distally relative to the outer shaft as indicated by the arrow in Fig. 3A, or the outer shaft may be retracted proximally relative to the inner shaft, or a combination of both motions may be used to remove the compression on the filaments thereby radially collapsing the tubular body into the collapsed configuration. The collapsed configuration has a small enough profile to permit delivery to the target treatment area (e.g. through a blood vessel lumen or another body channel). The diameter of the expanded device is determined by the geometry of the superimposed helical structure and may be any diameter, including but not limited to a diameter ranging from 2 to 35 millimeters. Similarly, the profile of the collapsed device maybe any size, including but not limited to 1.4 mm to 3.2 mm in diameter.

Thus, the tubular body may be expanded or collapsed to any size and provide any desired radial force from the fully collapsed configuration to the full expanded configuration and positions in between. Additionally, the device may expand and collapsed multiple times without fatiguing.

Fig. 4A shows an expandable scaffold 400 which may be any of the examples disclosed herein but with the proximal and distal ends of the filaments coupled to the inner 401 and outer shafts 404 asymmetrically. For example, in the example of Fig. 1, the helical filaments may be evenly distributed circumferentially around the inner and outer shafts to form a symmetrical cylindrical tube when expanded. However, in Fig. 4A, the distal ends of the filaments are coupled to an upper portion 403 of the inner shaft while the lower portion of the inner shaft is free of helical filaments. The proximal ends of the filaments are coupled to a lower portion 402 of the outer shaft while the upper portion of the outer shaft is free of filaments. The proximal and distal ends of the filaments may be offset any amount, but in Fig. 4A, the ends are offset approximately 180 degrees circumferentially from one another relative to the longitudinal axis of the inner and outer coaxial shafts. The middle section of the helical filaments between the proximal and distal filament ends may be evenly distributed around the inner and outer shafts to form a tubular body.

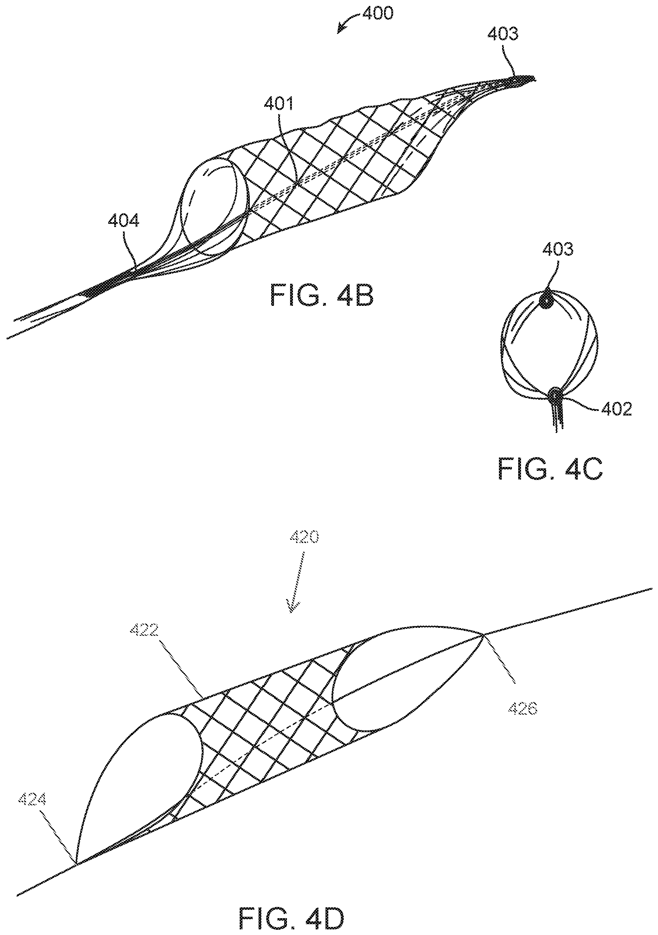

Fig. 4B shows the expanded tubular body formed when the example of Fig. 4A is radially expanded. The tubular body has a central portion that is substantially a cylindrical tube symmetric in shape, while the distal end has a distally tapering pointed end and the wall of the tubular body tapers from a full closed cylindrical wall to a partial open wall that tapers down to a point where the filaments are coupled to the inner shaft 401. And similarly, the proximal end also tapers proximally down from a full cylindrical closed wall to a partial open wall that tapers down to a point where the filaments are coupled to the outer shaft 404. This may also be described as the partially open wall flaring distally to the fully closed cylindrical wall. In this example, the proximal and distal coupling points are offset approximately 180 degrees from one another. The tapered proximal and distal open walls are pointed protrusions that are offset by approximately 180 degrees from one another. Any offset may be established by adjusting the proximal and distal connection points of the filaments to the inner and outer shafts.

Fig. 4C shows the proximal and distal connection points where the helical filaments are coupled to the inner shaft and the outer shaft circumferentially offset from one another by approximately 180 degrees, although this is not intended to be limiting and the offset may be set to any value between 0 and 360 degrees relative to the first connection point.

Fig. 4D shows another example of an expandable tubular body 420 similar to Figs. 4A-4B, but with the major difference being the proximal and distal openings are offset from one another, here the offset is approximately 90 degrees. The expandable tubular body 422 is generally the same as the example of Figs. 4A-4B, with the tubular body being formed form a plurality of helical wire filaments that form a cylindrical tube with a lumen extending from the proximal to distal ends of the tube. The filaments converge toward a point 424 on either the inner shaft or the outer shaft to form a proximal opening that is tear drop or bevel shaped and that lies in a first plane. The filaments on the distal end of the tube converge toward a point 426 on the other of the inner shaft or the outer shaft to form a distal opening that is also tear drop or bevel shaped that lies in a second plane. Here, the second plane is rotated by about 90 degrees relative to the first plane. Of course, by adjusting the circumferential offset between connection points 424, 426 on the inner and outer shafts, the proximal and distal openings may also be offset from one another to any desired amount, such as 15, 30, 45, or ninety degrees or more. The inner and outer shafts may be disposed in the lumen of the expandable member, or the shafts may lie against an inner or outer surface of the expandable member so that they do not obstruct the lumen. Operation of the tubular member is substantially the same as described previously in Figs. 4A-4C above. Forming the large proximal and distal openings allow another therapeutic or diagnostic device to be inserted through the expandable tubular body after it has been expanded and supports adjacent tissue.

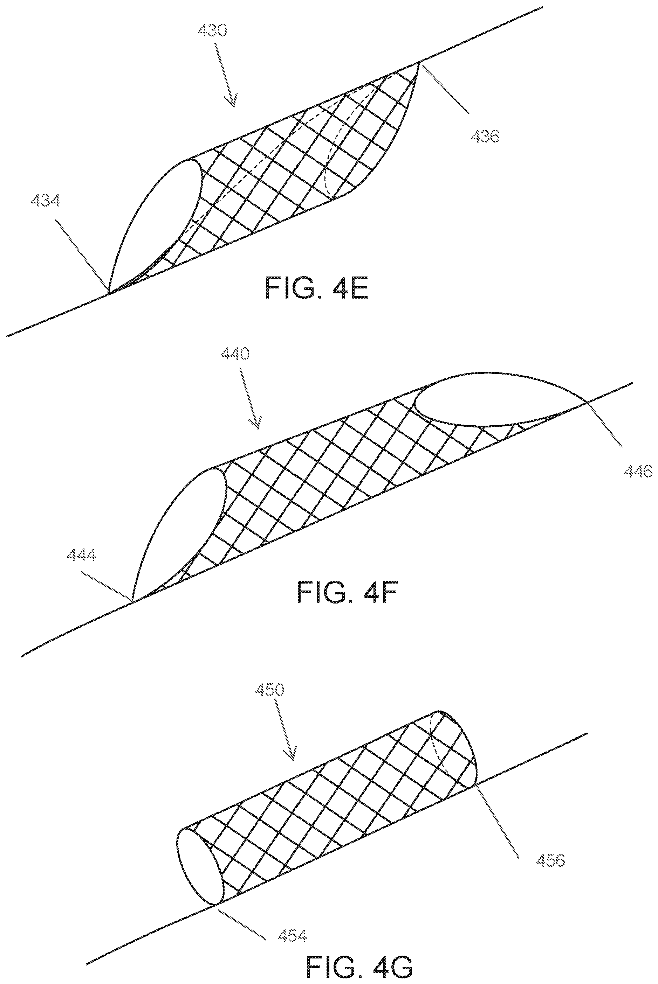

Fig. 4E shows another example of an expandable tubular body 430 that is generally the same as the example of Fig. 4D with the major difference being that the proximal and distal connections points 434, 436 are now circumferentially offset by about 180 degrees so the openings lie in planes that are parallel with one another. The openings are again tear drop or bevel shaped, although the pointed portions of the tear drop shaped opening are opposite of one another with one facing downward and one facing upward. Other aspects of the device in Fig. 4E are generally the same as previously disclosed with respect to Figs. 4A-4E.

Fig. 4F shows another example of an expandable tubular body 440 that is generally the same as the example of Fig. 4E with the major difference being that the proximal and distal connection points 444, 446 are not circumferentially offset but longitudinally offset, and they both connect to an upper point on either the respective inner or outer shafts forming proximal and distal openings. The proximal opening is again a tear drop or bevel shaped opening that lies in a first plane, and the distal opening is also a tear drop or bevel shaped opening that lies in a second plane that is transverse to the first plane. The pointed portion of the tear drop is adjacent the inner or outer shaft. Other aspects of the device in Fig. 4F are generally the same as previously described with respect to Figs. 4A-4F.

Fig. 4G shoes another example of an expandable tubular body 450 that is generally the same as the example of Fig. 4E with the major difference being that the proximal and distal connection points 454, 456 are not offset circumferentially. They are longitudinally offset and they both connect to an upper point on either the respective inner or outer shafts forming proximal and distal openings. Here, the expandable tubular body is cylindrical and the openings are circular as they lie in planes that are substantially perpendicular to the longitudinal axis of the device, therefore both proximal and distal openings are circular and lies in generally planes that are parallel to one another. Other aspects of the device in Fig. 4G are generally the same as previously described with respect to Figs. 4A-4F.

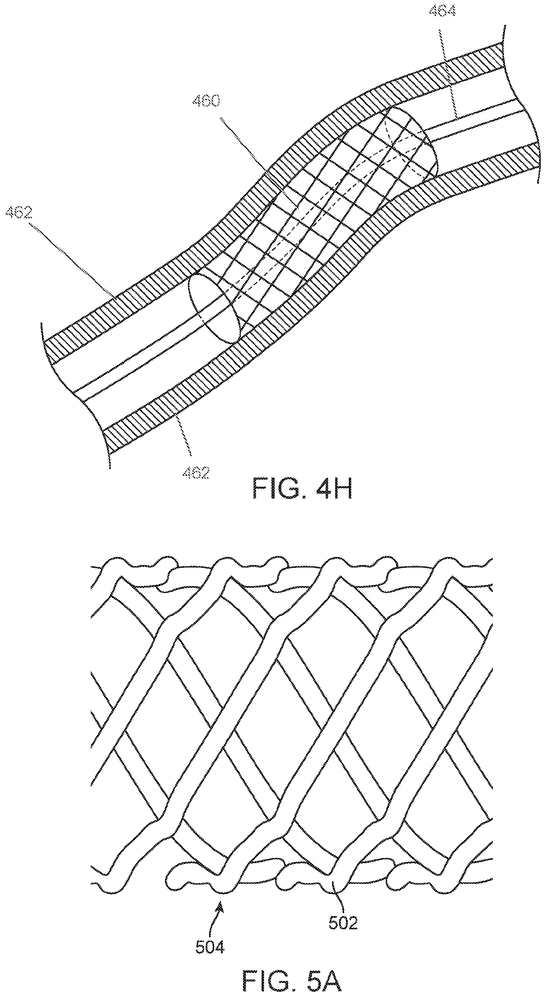

Fig. 4H shows an example of passing another therapeutic or diagnostic device 464 through an expandable tubular body 460 which may be any of those disclosed herein. After the expandable tubular body 460 has been delivered to the target treatment area and expanded to support tissue 462 such as a vessel wall, the second therapeutic or diagnostic device 464 may be passed from the proximal opening, through the lumen and out the distal opening of the expandable device 460 to a desired location.

The examples of Figs. 4A-4H illustrate that the use of asymmetrical coupling of the proximal and distal ends of the filaments to the inner and outer shafts allow other devices to cross the temporarily implanted expandable member so that additional diagnostic or therapeutic procedures may be performed adjacent to or distal of the expandable member. Any of the examples of expandable tubular members may have any of the wire mesh or weave patterns disclosed herein.

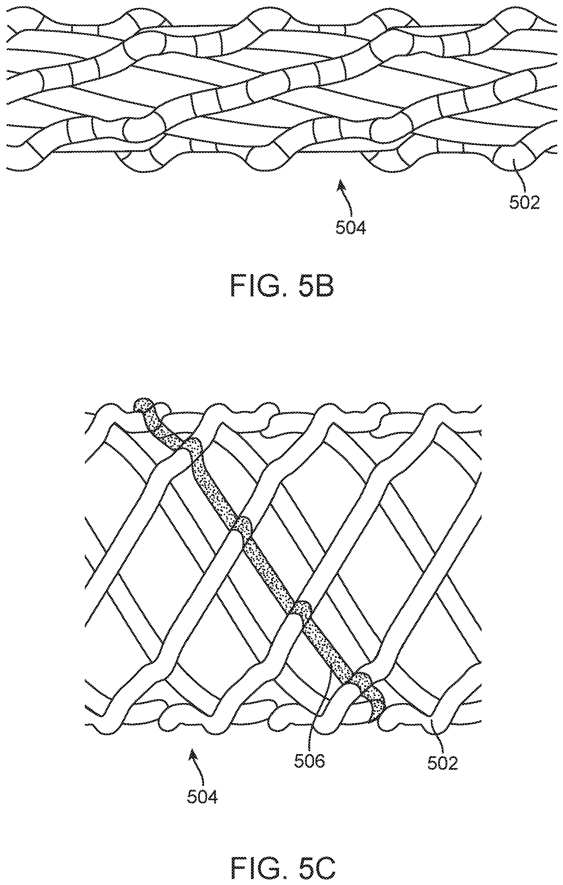

Figs. 5A-5D illustrate various examples of superimposed helical filaments 502. Fig. 5A shows the tubular body 504 formed by the superimposed helical filaments 502 in an expanded configuration, and Fig. 5B shows the tubular body of Fig. 5A in the collapsed configuration. In Fig. 5A the tubular body is formed from a plurality of superimposed helical filaments. At no position are there more than two filaments, so there is an inner-most layer of filaments and an outer-most layer of filaments. Thus, the tubular body has a wall that is a single layer formed by no more than two filaments stacked on top of one another.

Additional details about how the filaments are stacked on top of one another are disclosed below. Also, in the example of Fig. 5A, the filaments are always either on top of another filament or underneath a filament. The filaments never interweave with one another, so a filament is never on top of another filament in one position and then under the same filament at a different position.

Also, in Fig. 5A, the closed diamond-shaped cell formed by the helical filaments is elongated in the circumferential direction such that the distance from peak to peak or valley in the circumferential direction is longer than the distance from peak to peak or valley in the longitudinal direction. In the collapsed configuration, this changes so that the peak to peak or valley distance in the longitudinal direction is greater than the peak to peak or valley distance in the circumferential direction. Additionally, the angle formed by the crossing filaments is greater in the expanded configuration and smaller in the collapsed configuration. The filaments may cross one another so that a slightly raised thickness exists at the crossing point of two filaments while the portion of the filaments that do not cross are in the same circumferential plane as the other filaments, or slightly raised in a second plane as will be disclosed in greater detail below. Of course, one of skill in the art will appreciate that any number of open or closed cell geometries may be used and therefore diamond-shaped cells are not required or limiting in this or any of the examples disclosed herein. Other examples of cells may be square, rectangular, oval, elliptical, combinations thereof, or any other shape, and the filaments may also have any configuration including but not limited to linear, curved, undulating, combinations thereof, or any other shape.

Fig. 5B shows the tubular body of Fig. 5A in the collapsed configuration.

Fig. 5C illustrates how an additional filament 506 may be added to the example of Figs. 5A-5B. Here, the additional filament 506 (highlighted in a darker line) remains disposed on top of each filament that it crosses and the point where two filaments crosses is slightly deformed so that the portion of the filament that does not cross another filament is in the same circumferential plane as the other filaments. The portion of the filament that crosses another filament may be slightly raised or other configurations are possible as will be discussed below. In this example, the tubular body is formed from an inner-most layer of filaments, a middle layer of filaments, and an outer-most layer of filaments. Therefore, the tubular body has a wall that is a single layer formed by no more than three filaments stacked on top of one another. Additionally, the inner-most layer of filaments maybe aligned in one helical direction, the middle layer transverse to the inner-most helical direction, and the outer-most layer of filaments transverse to the middle helical direction (or substantially parallel to the inner-most helical direction).

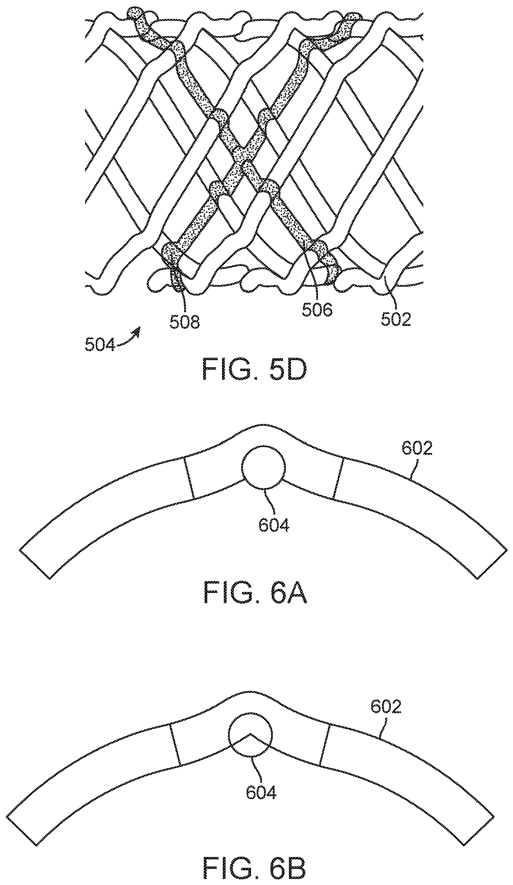

Fig. 5D adds yet another layer of filaments 508 to the example in Fig. 5C so now there are four layers of filaments that only cross one another so only two filaments are coupled together at any crossing point. And in this example, there is an inner-most layer of filaments, an inner middle layer of filaments, and outer middle layer of filaments, and an outer-most layer of filaments. Again, the filaments may alter their helical orientation from layer to layer so that one layer is transverse to the adjacent layer. The filaments maybe stacked on top of one another in any manner such as previously described above, or in any of the stacking examples described below.

Regardless of how many additional wires are superimposed, the sections of the wires between superimposed cross-sections result in a single layer of helical wires adjacent to the tissue, or bodily lumen (vessel, airway). Regardless of how many superimposed cross-sections of helical wires are present, the superimposed sections are directed toward the vessel wall or the airway wall. Each superimposed cross-section of helical wire may have a separate semi-flexible joint as discussed below.

Figs. 6A-6C illustrate various examples of how the filaments may cross an adjacent filament and this may be used in any of the examples of radially expandable devices disclosed herein.

Fig. 6A illustrates an example of two filaments 602, 604 that cross one another in any of the examples of radially expandable devices disclosed herein. The lower filament 604 is shown with a circular cross section that is undeformed. The upper filament 602 is shown crossing the lower filament such that the upper filament is slightly deformed around the lower filament to keep the total thickness of both filaments less than equal to the thickness of both filaments combined. Similarly, the thickness of the wall of the tubular body formed by both filaments will have a thickness that is also less than or equal to the thickness of both filaments combined. The filaments 602, 604 may be coupled to one another at the crossing point without any joint formed between the filaments, or the filaments may be joined together with a weld, adhesive, solvent bonded, or using any other techniques known in the art to form a joint. The joint may be rigid or resilient as will be discussed in greater detail below.

Fig. 6B shows the same filaments 602, 604 as in Fig. 6A overlapping one another to form a crossing point which may be used in any example of tubular body herein. However, in this example, the lower filament 604 is partially fused with the upper filament 602 to form the joint. The joint may be formed by welding, solvent bonding, etc. In this example, the thickness of the crossing point will be less than or equal to the combined thickness of both filament and greater than the thickness of either filament alone. By fusing the filaments together, the overall thickness of the tubular body will have a lower profile than if the filaments are simply stacked on top of one another. The resulting joint may be rigid or resilient as will be described below.

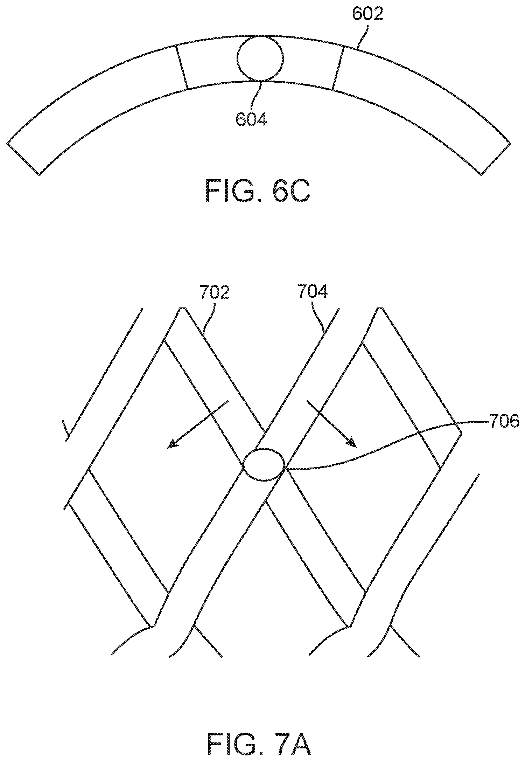

Fig. 6C shows another example of the crossing point formed by two filaments 602, 604 overlapping one another and which may be used in any tubular body example herein. Here, the two filaments are fully fused together so that the thickness of the joint or crossing point is less than or equal to the thickness of the upper filament 602. Again, the fusing maybe accomplished by any technique known in the art including welding, solvent bonding, etc. This configuration results in the lowest profile of the crossing point and the joint may be rigid, flexible or semi-flexible as discussed below.

**In** any of the examples shown in Figs. 6A-6C, the filaments may have a round cross-section or any other cross-sectional shape including square, rectangular, elliptical, oval, etc. Additionally, as mentioned, the resulting joint between overlapping filaments may be a rigid joint or it may be a resilient joint that is fully or semi-flexible and configured to move as the tubular body is expanded and collapsed while still constraining axial movement of the filaments relative to one another. Thus, the filaments can pivot or rotate relative to one another but they do not linearly slide relative to one another. The thickness of the semi-flexible joint can range from the diameter or thickness of a single filament d₁, or superimposed filament d₂ and the sum of the two filaments d₁ + d₂. The filaments may all have the same cross-section and thus same diameter or thickness, or they may be different. Similarly, they may be the same material, or different materials or hybrid materials. Examples of metallic materials that may be used for the filaments include but are not limited to stainless steel and metal alloys of nickel and titanium (e.g. nitinol materials). The filaments maybe formed with polymeric materials such as synthetic polymers, e.g. polyethylene terephthalate, polyamide, polyurethane, bioresorbable polymers such as polylactic acid (PLA), polyglycolic acid (PGA), polycaprolactone (PCL), polyanhydrides, poly(β-hydroxybutyrate), polydioxanone, poly(DTH iminocarbonate), polypropylene fumarate, etc. as well as copolymers thereof and mixtures thereof. In the hybrid device, both basic materials (metallic and polymeric) may be present in any proportion.

One filament cross-sectional size may be larger than another filament cross-sectional size, or smaller. Filament thickness may be determined based on design considerations such as radial strength and profile of the device in operation.

The expandable tubular body may have one joint or more than one joint. And the joints may be all resilient joints, all rigid joints, or combinations of resilient and rigid joints.

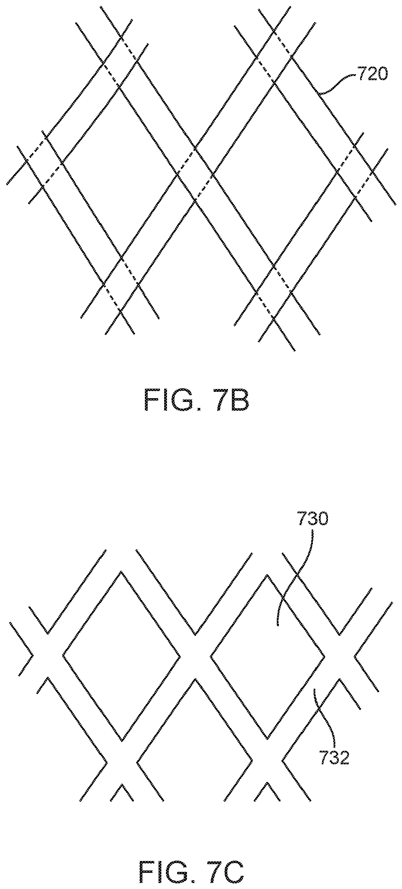

Fig. 7A shows the filaments forming a series of diamond shaped cells and the crossing point of two filaments 702, 704 which may be any filament described herein forming a joint 706 between the filaments. As disclosed above, this example and any other examples disclosed herein are not limited to diamond shaped cells. The cells may be open or closed, and may have any number of shapes including but not limited to rectangular, square, oval, elliptical, combinations thereof, or any shape. As previously discussed, the joint 706 may be simply the two filaments crossing and touching one another but unattached to one another, or the filaments may be attached to one another either fully fused together or partially fused together. The resulting joint may be rigid and inflexible, or the joint may be resilient and semi-flexible or fully flexible while maintaining the coupling of the overlapping filaments together. By joining the filaments together, the filaments are at least partially or fully constrained from sliding relative to one another thereby providing greater radial strength to the tubular body in the expanded configuration. A flexible or semi-flexible joint also allows the filaments to rotate or pivot relative to one another during expansion or collapse to reduce collapsed profile and still maintain radial strength as well as reducing the force required to radially expand the tubular body. Fig. 7A also shows how filaments 702 and 704 move away from one another as indicated by the arrows during collapsing of the tubular body. Therefore, the angle between the filaments will increase. The angle may be any angle between the filaments, but in this example may be the angle formed between adjacent filaments at a circumferentially oriented peak or valley of a closed cell. During expansion of the device, the filaments will move toward one another (in the opposite direction of the arrows) and the angle between filaments will decrease. The angle between adjacent filaments may move between 0 degrees and 90 degrees during expansion and collapse of the device. Similarly, for the angle formed between adjacent struts forming a peak or valley in the longitudinal direction, the angle decreases during collapsing and increases during radial expansion. Thus, a flexible or semi-flexible joint provides more desirable mechanical properties to the device as compared to an example where the filaments are woven together such that one filament passes on top of another filament and then goes under the next filament allowing the filaments to slide relative to one another during expansion and contraction as discussed below with reference to Fig. 7B.

Fig. 7B shows an example where the filaments 720 are woven with one another so that a filament is disposed under one filament and then disposed over the adjacent filament in a repeating pattern. This may not provide as much radial strength as the joints described above in Fig. 7A because the filaments are unconstrained and free to slide relative to one another during expansion and collapsing of the device. Additionally, the profile of the device will be larger due to the overlapping filaments.

Fig. 7C shows a radially expandable tubular member that is formed from a plurality of slots 730 cut from a tube such as hypotube. The slots expand into diamonds during radially expansion and the slots flatten out during collapsing. Because the struts 732 are integral with one another this configuration provides radial strength but may require greater expansion forces to move the struts during expansion since there is no flexible or semi-flexible joint as seen with the superimposed helical filament examples disclosed herein, and the material around the joints must bend either elastically or plastically. This also makes it harder to uniformly collapse the tubular body once it is radially expanded, and repeated expansion and collapsing may fatigue and break the struts. Thus, the example of Fig. 7A provides many of the advantages of both the woven configuration of Fig. 7B and the integral strut or filament design of Fig. 7C, thereby providing relatively easy expansion and collapsing as well as good radial strength.

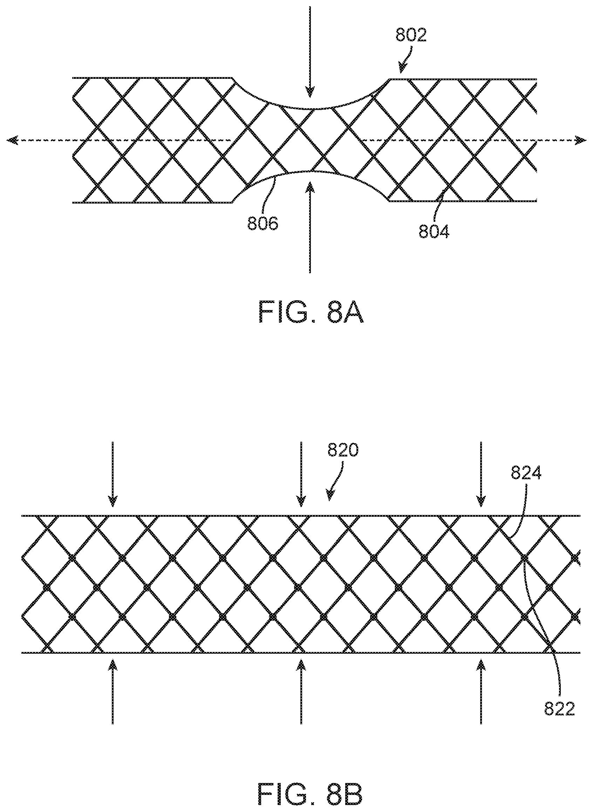

Fig. 8A shows a typical woven design for a radially expandable member 802 where the wires extend under one adjacent wire and over another adjacent wire such as previously described in Fig. 7B above, and will be compared to the superimposed helical filament design in Fig. 8B which includes joints between the overlapping filaments. Fig. 8B provides some advantages over Fig. 8A.

In Fig. 8A the wires 804 are free to move relative to one another and thus radial strength in the expanded configuration may be reduced and the expanded tubular body may not have adequate radial strength. When a force as indicated by the inwardly pointing arrows is applied to the device, if the radial strength of the tubular body is not strong enough, the body will collapse 806 radially inward either at a discrete location or all along the tubular body, depending on how the force is applied to the tubular body. If the tubular body collapses it will not provide adequate support to the tissue and may not remain in apposition with the tissue thereby preventing or hindering therapeutic agent to be delivered to the tissue.

Fig. 8B shows a force (as indicated by the apposed arrows) applied to a tubular body 820 with adequate radial strength. Because the radial strength is greater than the force applied to the tubular body, the tubular body does not collapse and maintains its shape and continues to support and engage the tissue. The helical filaments 824 and the flexible or semi-flexible joints 822 allow the device to have uniform radial strength along its longitudinal axis, and that is greater than a traditionally woven tubular body. The tubular body 820 in Fig. 8B may be any of examples of superimposed helical filament examples disclosed herein.

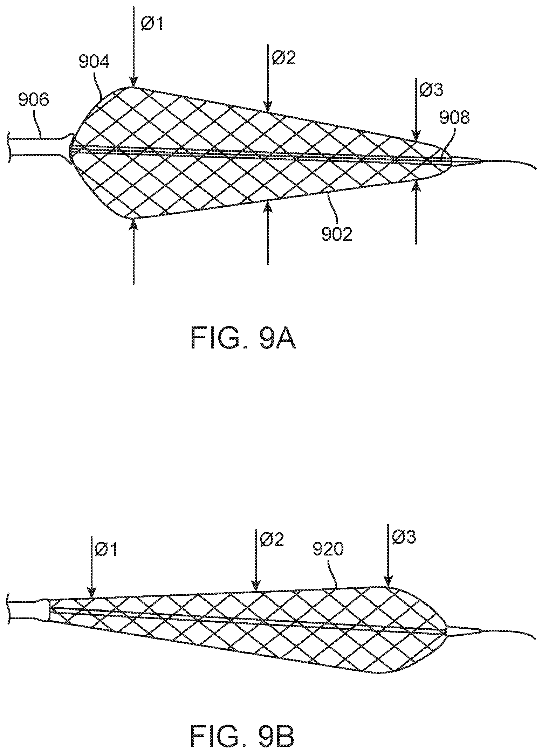

Figs. 9A-9D illustrate various geometries of radially expandable tubular bodies that may be formed using the helical filament construction described above. An optional guidewire is shown extending from the distal tip of the device.

Fig. 9A shows a radially expandable tubular body 902 that tapers from the proximal end to the distal end. Diameter Θ₁ is greater than diameter Θ₂ which is greater than diameter Θ₃. Therefore, a brief shoulder 904 is coupled to the outer shaft 906 and then the maximum diameter is shown at the proximal end of the tubular body and the diameter tapers gradually down to the minimum diameter at the distal end which is coupled to the inner shaft 908. Tapering allows the radially expandable body to conform with the walls of a body channel such as a vascular lumen that has a similar taper in the proximal to distal direction. Actuation of the tubular body functions generally the same way as previously described above in Figs. 3A-3B.

Fig. 9B shows an example similar to Fig. 9A with the major difference being that the taper is reversed and therefor the maximum diameter is near the distal end of the tubular body and this tapers proximally to the small diameter on the proximal end, thus the tubular body 920 has a diameter Θ₁ that is smaller than diameter Θ₂ which is smaller than diameter Θ₃. Again, this taper allows the expandable member to conform to the walls of a body channel such as a vascular lumen that has a similar taper in the distal to proximal direction. This example may also be described as having a distal flared tubular body.

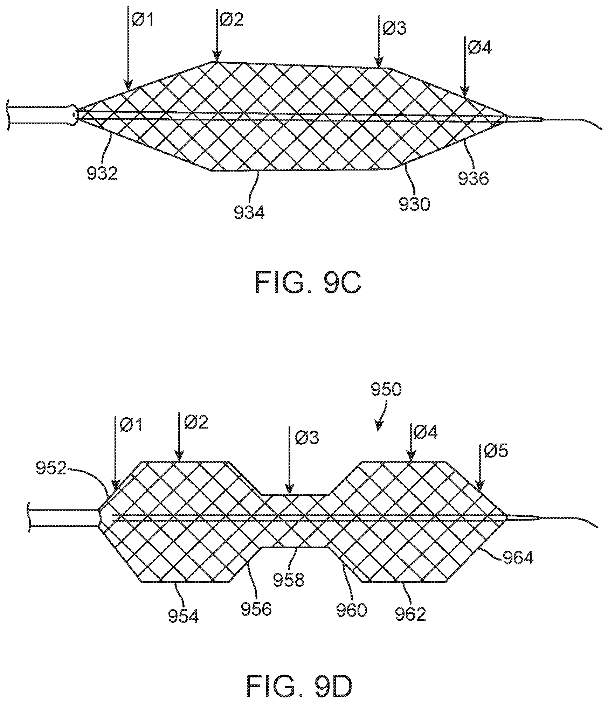

Fig. 9C shows an expandable member 930 having a flared proximal 932 end with increasing diameter, then a constant diameter mid-section 934 that then transitions into a distally tapering region 936. Thus, proximal diameter Θ₁ is smaller than diameter Θ₂ which is the same as diameter Θ₃ which is greater than Θ₄. The constant diameter mid-section 934 may be the working portion that is used to support tissue and the flared and tapered should sections 932, 936 may not support or engage tissue. Or the body channel may have walls which match this shape and are supported by some or all sections of the flared, constant diameter or tapered sections.

Fig. 9D shows still another example of an expandable tubular body 950 that may be formed from any of the helical filament examples disclosed herein. In this example, the tubular body is formed from helical filaments with any of the joints described herein. A proximal section has a flared region 952, then the tubular body has a constant diameter section 954 followed by a taper 956 that tapers distally to a center section 958 that has a constant diameter. The constant diameter center section then flares outward 960 again increasing in diameter to another constant diameter section 962, followed by a distally tapering section 964. Again, any shape may be formed in order to support the target treatment tissue. Thus, diameter Θ₁ is smaller than diameter Θ₂ which is larger than diameter Θ₃ which is smaller than diameter Θ₄ which is larger than diameter Θ₅.

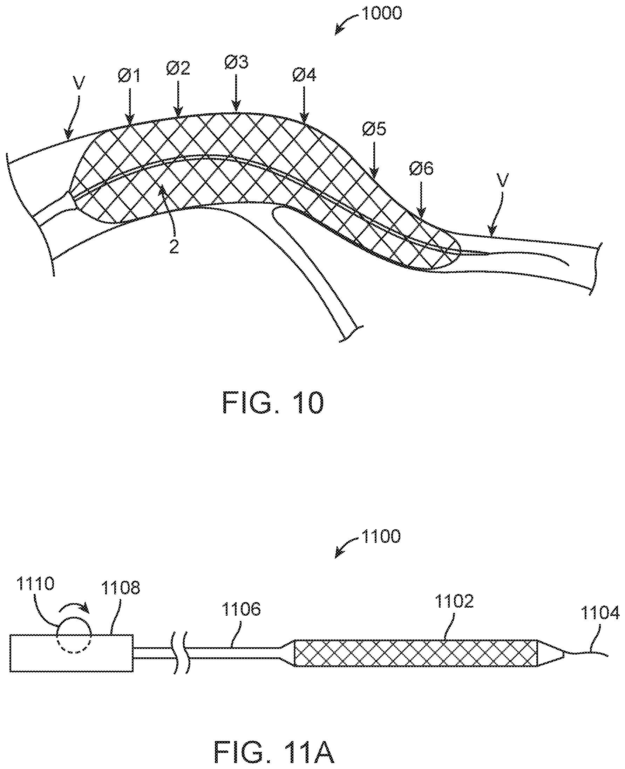

Fig. 10 shows how a superimposed helical filament design 1000 such as any of those disclosed herein may be used to provide a personalized design that adapts to the local anatomy. Because the filaments may have a resilient joint coupling overlapping filaments, the outer diameter of the expanded tubular body may expanded until it engages the treatment tissue, here a blood vessel V, and thus the expanded tubular body will conform to the contours of the treatment region so that diameter and curvature of the tubular body may vary along its length. Thus, diameters Θ₁, Θ₂, Θ₃, Θ₄, Θ₅, and Θ₆ may be the same, may be different than one another or some may be the same and some different depending on the diameter of the vessel V. This customized expandable device therefore adapts to the anatomy based on medical information and imaging such as angiograms, computerized tomography (CT), computerized tomography angiography (CTA), magnetic resonance imaging (MRI), magnetic resonance angiography (MRA), ultrasound, x-ray, optical coherence tomography (OCT), intravascular ultrasound (IVUS), as well as any other imaging modality known in the art, and provides a variable diameter tubular body that conforms to a vessel wall or any other tissue wall. Here the proximal end of the tubular body flares outward with increasing diameter to a maximum diameter section which then gradually tapers distally to the distal end. The tubular body is expanded and in engagement with the walls of a vessel to support the vessel and/or elute a therapeutic agent to the tissue that engages the tubular body. In addition, the diameter of the device conforms to the walls of the treatment site, the longitudinal axis of the device also conforms to the curvature of the channel, vessel or other body cavity. A drug may be delivered from the expandable tubular body and/or the device may simply provide temporary support to the walls of the tissue until they can remodel and remain patent. The helical filament construction with flexible or semi-flexible joints also allows the tubular body to provide a uniform radial outward force on the tissue even though the diameter varies.

Fig. 11A shows an example of a support device 1100 having an expandable tubular body 1 102 which may be any of those disclosed herein formed with any of the helical filament examples herein and having any of the filament joints disclosed herein. Optional guidewire 1104 extends past the distal end of the catheter. Inner and outer shafts 1106 (only the outer shaft is visible in this view) are coupled to the expandable member 1102 as described previously and are used to expand and collapse the radially expandable member. The proximal ends of the inner and outer shafts are operatively coupled to a handle 1108 that is ergonomically shaped to allow an operator to grasp and manipulate the handle. The handle may include an actuator 1110 such as a slide, lever, rotatable wheel or other actuation element that is operatively coupled to the inner and outer shafts such that actuation of the actuator in one direction will move the inner and outer shafts relative to one another to radially expand the tubular body, while actuation of the actuator in the opposite direction will move the inner and outer shafts in an opposite direction relative to one another thereby collapsing the radially expandable member. The handle may also include a gage or other visual indicia (not shown) to show the operator the diameter of the expandable tubular body. An optional guidewire 1104 is shown slidably disposed in the device.

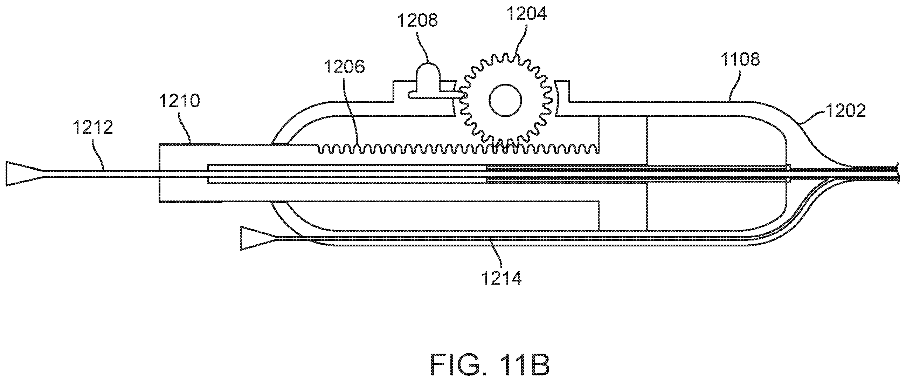

Fig. 11B shows an example of a handle 1108 which may be used in Fig. 11A or with any of the examples disclosed herein. Handle 1108 includes a housing 1202 which is ergonomically shaped to fit in an operator's hand and houses at least some of the actuator components including an actuator wheel 1204, a rack 1206, and a locking element 1208. The inner shaft 1212 may be coupled to a plunger 1210 that is coupled to the rack 1206. The rack includes a number of teeth that engage with the teeth on actuator wheel 1204. Thus, as actuator wheel 1204 is rotated in one direction, the rack will move in one direction and the inner shaft will therefore also move in one direction. When the actuator wheel is rotated in a second opposite direction, the rack will move in a second opposite direction thereby causing the inner shaft to move in a second opposite direction.

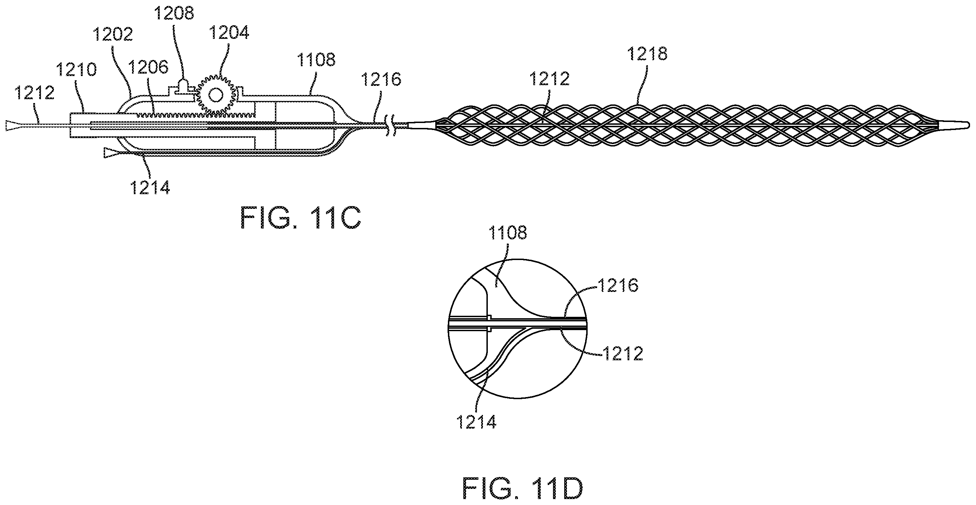

Fig. 11C shows the handle of Fig. 11B coupled to an expandable member 1218 which may be any of those described herein. The outer shaft 1216 is fixed to the handle, thus resulting in relative movement between the inner and outer shafts which can therefore expand or collapse the expandable member 1218. The wheel may be actuated multiple times back and forth to expand and collapse the expandable member as needed. The inner shaft may extend to the proximal end of the handle where a valve, Luer connector or other connector may be coupled to the proximal end of the inner shaft to prevent fluid from flowing past the proximal end. A guidewire (not illustrated in this figure) may be inserted into a lumen of the inner shaft and extend all the way through the inner shaft lumen and exit at the distal end of the device as shown in previous figures, and the lumen may also be flushed with a fluid to remove air or other unwanted fluids or gases. As indicated earlier, the outer shaft is fixed to the handle and therefore is stationary in this example. The annular gap between the inner and outer shafts may also be flushed with a second path 1214 that is fluidly coupled to the annular gap. The proximal end of pathway 1214 may also have a Luer connector, stopcock, or any other connector as needed to control fluid flow.

As the radially expandable member is expanded or collapsed, it may be desired to lock the diameter at a certain size and hold it at that size during a procedure. A lock 1208 may be included on the handle. The lock can be any number of locks such as a pawl that prevents movement of the actuator wheel unless the lock is unlocked.

Fig. 11D highlights a distal section of the handle in Fig. 11C and illustrates how the flush lumen 1214 is coupled to the annular space between the inner and outer shafts 1212, 1216.

And this example is not intended to be limiting. As discussed previously, relative movement of the inner and outer shafts is needed to expand and collapse the expandable member. Therefore, the outer shaft may be coupled to the rack and the inner shaft maybe fixed to the handle in another example. Other actuators are known in the art and may also be used. Levers, slides, or any other known actuators may be used instead of an actuator wheel. The actuator wheel or other actuator may also have indicia that indicate the diameter or size of the expandable member as it expands and collapses. For example, the actuator wheel may have a clicking noise to indicate size, or markings which show the diameter.

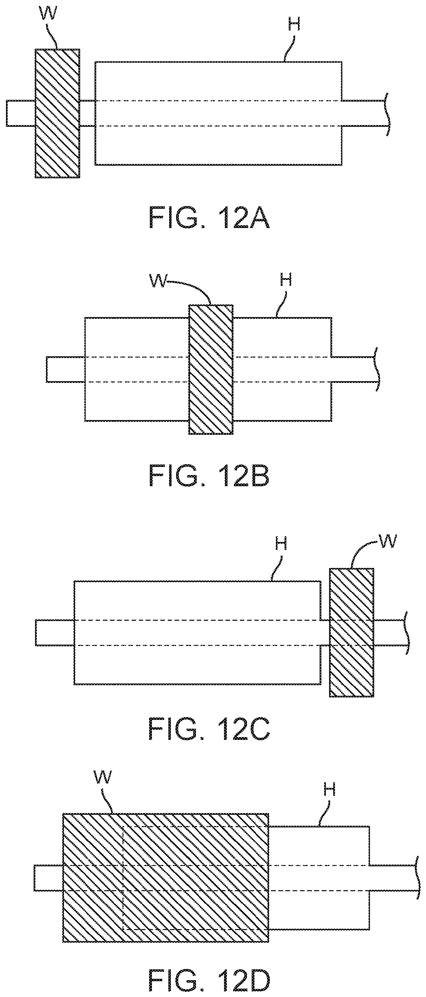

Figs. 12A-12I illustrate various examples of actuator orientations relative to the handle that may be used in any example. The handle H is coupled to the inner and outer shafts as previously described above. An actuator, here a rotatable wheel W is shown coupled to the handle H.

In Fig. 12A, the wheel W is on a proximal end of the handle H and the wheel is transverse or orthogonal to the longitudinal axis of the handle. The wheel rotates around the longitudinal axis of the handle and inner/outer shafts.

Fig. 12B shows a similar example as Fig. 12A with the major difference being that the wheel W has been moved so it is now disposed over a middle portion of the handle H but otherwise is substantially similar to the example in Fig. 12A.

Fig. 12C shows a similar example as Fig. 12A with the major difference being that the wheel W has been moved so that it is now disposed at the proximal end of the handle H but otherwise is substantially similar to the example in Fig. 12A.

Fig. 12D illustrates an example where the handle H includes the rotatable wheel W. Here, a proximal portion of the handle is the rotatable wheel W and therefore as the handle is rotated in different directions, the handle will be drawn into or away from the rotatable wheel. The wheel may be concentric with the inner and outer shafts.

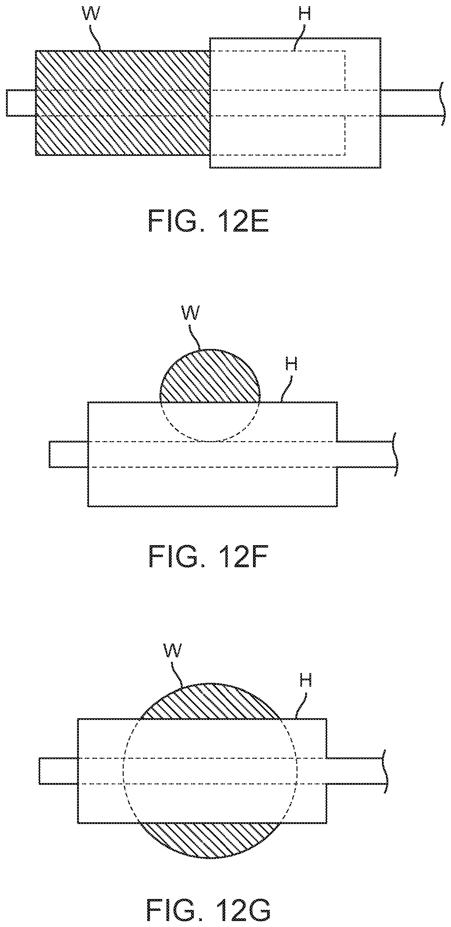

Fig. 12E is similar to the example of Fig. 12D with the major difference being that the handle body H has a larger size than the rotatable wheel. Rotation of the wheel will draw the wheel into or way from the handle. Rotation of the wheel is generally concentric to the longitudinal axis of the inner and outer shafts.

Fig. 12F shows the rotatable wheel W protruding from a top or bottom surface of the handle H. Approximately half of the wheel will be exposed while approximately the other half will be housed in the handle. The wheel may be located in the middle portion of the handle or anywhere on the handle and rotation of the wheel will generally be in the same parallel direction to the inner and outer shafts.

Fig. 12G shows another example of a wheel W that protrudes from the top and the bottom surfaces of the handle H. Therefore, the wheel may be actuated from either the top or bottom. Actuation is generally in the same direction as above in Fig. 12F.

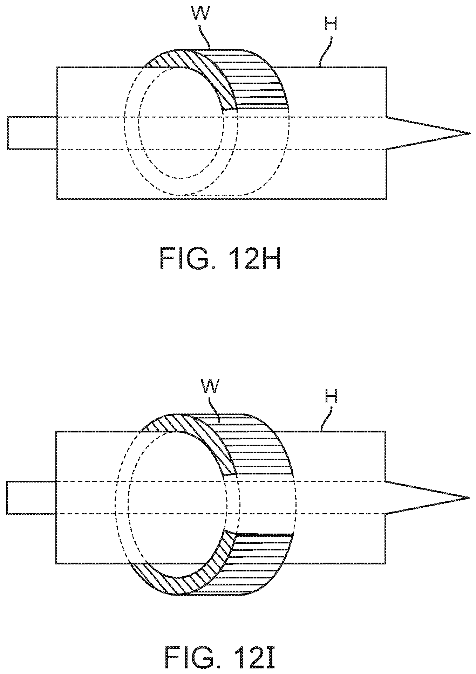

Fig. 12H shows another example of an actuator wheel W coupled to a handle H. Here the wheel W may be disposed outside the handle and is now canted relative to the handle and therefore the wheel rotates in a plane that is transverse or oblique to the longitudinal axis of the inner and outer shaft. Here, a left side of the wheel is canted proximally, while a right side of the wheel canted distally in this side view of the handle. The wheel may extend partially or completely around the handle to form a closed or open ring.

Fig. 12I is another example of a canted wheel W that is disposed around the outside of the handle H. Here the wheel will rotate in a plane that is transverse or oblique the longitudinal axis of the inner and outer shafts. In this example which is a side view of the handle, the top of the wheel is canted proximally and the bottom of the wheel is canted distally. The wheel may extend completely or partially around the handle to form a closed ring or an open ring.

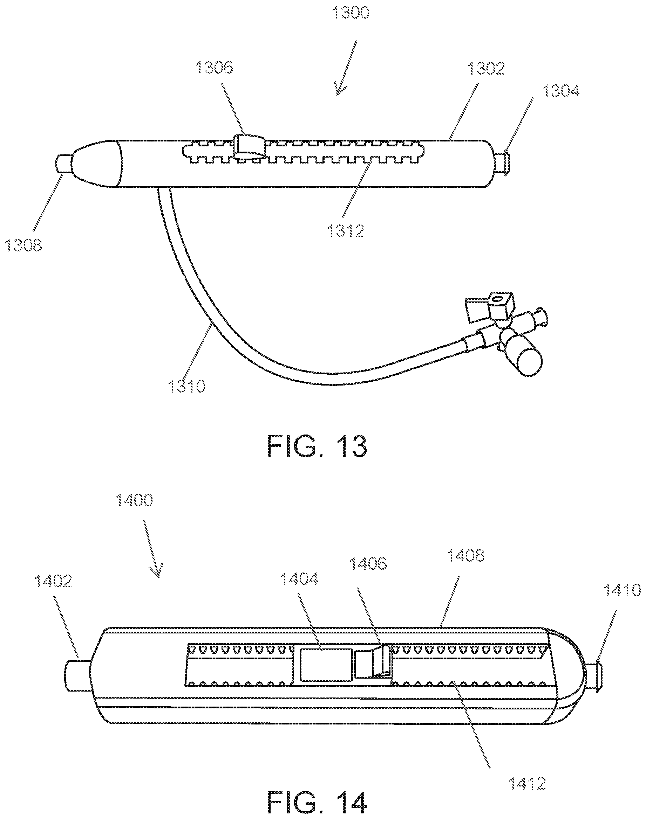

Fig. 13 shows another example of a handle 1300 that may be used with any of the expandable tubular member devices disclosed herein. The handle 1300 includes a housing 1302 which is ergonomically shaped to fit in an operator's hand. For example, the housing may be cylindrical, rectangular, square, or have other cross-sectional shapes. The actuator 1306 may be a slider mechanism that slides along a track 1312 with detents that provide discrete stops for the slider. Thus, the slider may be advanced or retracted a desired amount and once the slider locks into the adjacent detent, the slider position is held until the operator moves it again. Thus, any of the expandable members may be fully or partially opened and held in that position, and then the expandable member may be fully or partially collapsed and held in that position. The detents may be marked with colors or labels to indicate the size of the expandable member or the detents may click as the slider is moved along the track 1312 to provide audible feedback to the operator about slider position. The proximal end of the handle may include a connector such as a Luer connector 1304 to allow tubing, syringes or other devices to be releasably coupled to the handle, or to allow a guidewire to be advanced through the handle and through the inner shaft. The inner and outer shafts maybe operably coupled with the handle and slider. For example, if the inner shaft moves relative to the outer shaft as discussed previously above, one of the inner or outer shafts maybe coupled to the slider and the other shaft is coupled to the handle. Therefore, as the slider is actuated, it will move the respective shaft while the other shaft remains stationary. Only the outer shaft 1308 is illustrated in Fig. 13. An optional side port 1310 which includes a short length of tubing with an optional stopcock on the end may be coupled with a syringe or other tubing to allow fluid to be introduced into the annular space between the inner and outer shafts as discussed above in Figs. 11A-11D thereby flushing out entrapped air, or allowing a therapeutic or diagnostic agent to be introduced through the annular gap and out the distal end of the device.

Fig. 14 shows another example of a handle 1400 that may be used with any of the examples of expandable members disclosed herein. The handle includes a housing 1408 that may be any shape such as those described above in Fig. 13 and that are ergonomically shaped for an operator. Here, the actuator includes a slider mechanism having a lock mechanism 1404 and a slider 1406 that slide along a track 1412 with detents. The slider is a raised ramp surface that allows an operator's finger or thumb to easily advance or retract the slider 1406 along track 1412. The lock mechanism 1404 is a button that may be depressed to unlock the slider and allow the operator to slidably advance or retract the slider. When the lock mechanism is undepressed it engages detents in the track 1412 to prevent it from moving, thereby locking the slider in place. The slider may be coupled to either the inner shaft or the outer shaft and the other shaft is then coupled to the housing 1408 in order to allow relative movement of the inner shaft to the outer shaft. And thus, the expandable member may be fully or partially expanded and then held in position by the lock, or the expandable member maybe fully or partially collapsed and held in position. Fig. 14 only shows the outer shaft 1402 coupled to the housing. An optional Luer connector 1410 may be disposed on the proximal end of the handle to allow a syringe or tubing to be coupled to the handle, or to allow a guidewire to be inserted through the handle and out the distal end of the inner shaft.

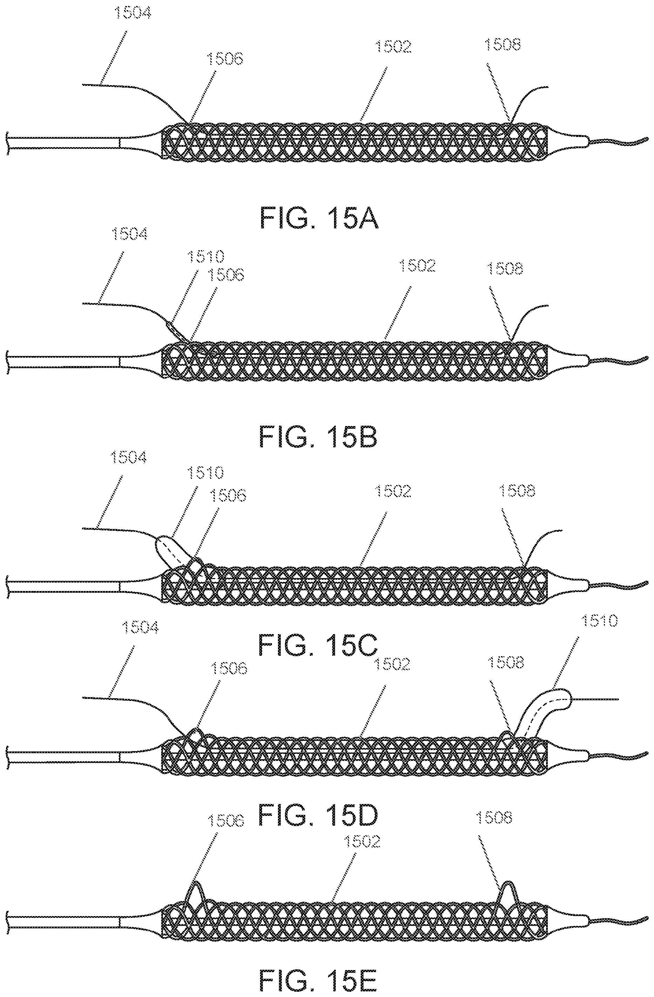

Figs. 15A-15E show an optional method that may be used with the example of expandable member shown in Fig. 1, or with any of the other examples of expandable members disclosed herein.

Fig. 15A shows the device 1502 which is the same as the device 10 shown in Fig. 1 in the expanded configuration. The wire filaments are coupled to the inner and outer shafts as previously discussed which closes the proximal and distal ends of the expandable member (except for the apertures formed by the wire filament pattern) and prevents an additional device from being passing through the lumen of the expandable member. It may be advantageous to allow a device to be passed through the expandable member therefore a guidewire 1504 may be passed through an aperture 1506 in the proximal side wall of the expandable member formed by the wire filament pattern. The guidewire is advanced distally through the lumen of the expandable member and then passed out of expandable member through an aperture 1508 in the distal side wall formed by the wire filament pattern.

In Fig. 15B, a balloon catheter 1510 or other expandable device is advanced over the guidewire 1504 and through the proximal aperture 1506. The balloon is in a collapsed configuration so that it may easily pass through the proximal aperture 1506 that is formed by the wire filaments.

In Fig. 15C the balloon 1510 or expandable member is radially expanded to increase the size of the proximal aperture 1506. The balloon may be expanded by filling it with a fluid such as saline or contrast media.

Referring now to Fig. Fig. 15D, the balloon is deflated and then advanced over the guidewire until it is disposed in the distal aperture 1508 that is formed by the wire filaments. The balloon is then expanded similarly with fluid such as saline or contrast media to enlarge the distal aperture. Once the desired aperture size is achieved with the expanded balloon, the balloon is deflated and then slidably retracted out of the distal aperture 1508 and removed from the device 1502. The guidewire 1504 is also removed.

Fig. 15E shows the device 1502 with enlarged proximal and distal openings 1506, 1508. A second therapeutic or diagnostic device may then be advanced through the proximal and/or distal apertures and through the expandable member 1502 as needed.

The procedure described in Figs. 15A-15E may also be applied to examples of expandable tubular devices that already have a proximal and distal aperture (e.g. Figs. 4A-4H) in order to further expand the existing proximal or distal apertures, or to create a new proximal or distal aperture.

Any example disclosed herein may include a therapeutic agent. The helical filaments or any portion of the device may be coated with a coating layer that contains a therapeutic agent or a mixture of therapeutic agents. The therapeutic agent may be carried by the helical filaments or any part of the device and may include paclitaxel, sirolimus also known as rapamycin, corticosteroids, nonsteroidal anti-inflammatory drugs, anticoagulants, etc. The therapeutic agent may be disposed in a coating coupled to the helical filaments such as a mixture of polymers and therapeutic agents. The polymers are drug carriers such as, polyvinyl alcohol, polyethylene glycol, polyvinyl polypyrrolidone, poly(L-lactide), polylysine, etc. The ratio of polymer to therapeutic agent may be any ratio but examples of this ratio may include: 1:9; 2:8; 3:7; 4:6, 5:5, 6:4 as well as any tenth of the ratio. For example, in a 1:9 ratio of polymer to therapeutic agent, the ratio may include any of 1.1; 1.2; 1.3; 1.4; 1.5; 1.6; 1.7; 1.8; or 1.9 to any of 9.1; 9.2; 9.3, 9.4; 9.5; 9.6; 9.7; 9.8; or 9.9.

In any example disclosed herein a fluid such as blood or any body fluid or any liquid, or a gas such as air is free to pass by the tubular body in the expanded or collapsed configuration because the tubular body has a lumen for fluid flow and the walls of the tubular body are porous. Additionally, the tubular body may be expanded and held in an expanded configuration for any length of time such as less than or equal to 30 seconds, or less than or equal to 30 minutes, or less than or equal to 60 minutes, or even longer than 60 minutes until a desired therapeutic effect is obtained. Desired therapeutic effects may include delivery of a therapeutic agent to the target treatment tissue or remodeling of the target treatment tissue so that it is self-supporting. Any of the examples of superimposed helical tubular bodies may be used to deliver therapeutic agents to a target treatment area. The therapeutic agent maybe delivered continuously over a short initial period of time, or during the entire time the device contacts the target treatment area. Moreover, the same device maybe used repeatably to expand and support one or different areas in the same patient as well as deliver a therapeutic agent in one or different areas in the same patient. Thus, any of the examples disclosed herein may be used to expand and collapse multiple times and be held in the expanded configuration for a desired time before being removed from the patient.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. The scope of the invention should be determined with reference to the appended claims.

## Claims

1. A temporary and expandable tissue support device, the device comprising:
a plurality of helical filaments (702, 704) superimposed on top of one another to form a tubular body having a first end, a second end opposite the first end, and a lumen extending between the first end and the second end;
an inner shaft (1212); and
an outer shaft (1216) slidably disposed over the inner shaft (1212),
wherein the first end is coupled to the inner shaft (1212), and the second end is coupled to the outer shaft (1216),
wherein actuation of the inner and outer shafts (1212 and 1216) in a first direction compresses the plurality of filaments (702, 704) thereby radially expanding the tubular body into an expanded configuration that is adapted to engage and support tissue at a treatment site without obstructing a fluid from flowing past the tubular body, and
wherein actuation of the inner and outer shafts (1212 and 1216) in a second direction opposite the first direction tensions the plurality of filaments (702, 704) thereby radially collapsing the tubular body into a collapsed configuration that is adapted to be delivered to or removed from the treatment site,
wherein the plurality of helical filaments (702, 704) each have a thickness and cross one another at a plurality of crossing points, and **characterised in that** each crossing point has a thickness greater than or equal to the thickness of one of the plurality of filaments, and wherein the thickness is less than or equal to the thickness of two of the plurality of filaments, wherein at least some of the plurality of crossing points comprises two of the plurality of helical filaments (702 and 704) fused together to form a resilient joint (706), wherein the resilient joint (706) is configured to flex as the tubular body moves between the expanded configuration and the collapsed configuration and the joined filaments are constrained from sliding relative to one other.

2. The device of claim 1, wherein each crossing point comprises no more than two of the plurality of helical filaments (702, 704).

3. The device of claim 1 or 2, wherein the plurality of filaments (702, 704) is disposed in a single circumferentially oriented planar layer.

4. The device of any of claims 1 to 3, wherein the two helical filaments (702, 704) form a crossing angle at the crossing point, and wherein the crossing angle changes up to 90 degrees during movement between the expanded configuration and the collapsed configuration.

5. The device of any of claims 1 to 4, wherein at least some of the plurality of crossing points comprises two of the plurality of helical filaments crossing one another without an attachment therebetween.

6. The device of any of claims 1 to 5, further comprising a therapeutic agent carried by the tubular body, wherein the therapeutic agent is configured to be delivered therefrom to the tissue at the treatment site.

7. The device of any of claims 1 to 6, further comprising a handle (1108) coupled to a proximal end of the inner shaft (1212) and a proximal end of the outer shaft (1216), the handle (1108) further comprising an actuator element (1204), wherein actuation of the actuator element (1204) moves the inner shaft (1212) relative to the outer shaft (1216)to expand or collapse the tubular body.

8. The device of any of claims 1 to 7, wherein the first end of the tubular body is coupled to the inner shaft (1212) asymmetrically or the second end of the tubular shaft is coupled to the outer shaft (1216) asymmetrically.

9. The device of any of claims 1 to 8, wherein the tubular body comprises a proximal opening (1506) and a distal opening (1508), and wherein the proximal and distal openings (1506 and 1508) lie in different planes.

10. The device of any of claims 1 to 9, wherein a fluid or a gas passes through or around the tubular body without restriction when the tubular body is in the expanded configuration or the collapsed configuration.

11. The device of any of claims 1 to 10, wherein the tubular body in the expanded configuration applies a constant force to the tissue.

12. The device of any of claims 1 to 11, wherein the tubular body has a first expanded configuration and a second expanded configuration, and wherein the first expanded configuration applies a force to the tissue, and wherein the second expanded configuration applies a second force greater than the first force to the tissue.

## Patentansprüche

1. Temporäre und ausdehnbare Gewebestützvorrichtung, wobei die Vorrichtung umfasst:
eine Mehrzahl von spiralförmigen Filamenten (702, 704), die übereinander angeordnet sind, um einen röhrenförmigen Körper auszubilden, der ein erstes Ende, ein dem ersten Ende gegenüberliegendes zweites Ende und ein sich zwischen dem ersten Ende und dem zweiten Ende erstreckendes Lumen aufweist;
einen Innenschaft (1212); und
einen Außenschaft (1216), der verschiebbar über dem Innenschaft (1212) angeordnet ist,
wobei das erste Ende mit dem Innenschaft (1212) gekoppelt ist und das zweite Ende mit dem Außenschaft (1216) gekoppelt ist,
wobei die Betätigung des Innen- und Außenschafts (1212 und 1216) in einer ersten Richtung die Mehrzahl von Filamenten (702, 704) zusammendrückt, wodurch der röhrenförmige Körper radial in eine ausgedehnte Konfiguration ausgedehnt wird, die angepasst ist, um an einer Behandlungsstelle in Gewebe einzugreifen und dieses zu stützen, ohne den Durchfluss eines Fluids an dem röhrenförmigen Körper vorbei zu behindern, und
wobei die Betätigung des Innen- und Außenschafts (1212 und 1216) in einer zweiten Richtung, die der ersten Richtung entgegengesetzt ist, die Mehrzahl von Filamenten (702, 704) spannt, wodurch sich der röhrenförmige Körper radial in eine zusammengefaltete Konfiguration zusammenfaltet, die angepasst ist, um an die Behandlungsstelle abgegeben oder von dieser entfernt zu werden,
wobei die Mehrzahl von spiralförmigen Filamenten (702, 704) jeweils eine Dicke aufweist und sich an einer Mehrzahl von Kreuzungspunkten kreuzt, und **dadurch gekennzeichnet, dass** jeder Kreuzungspunkt eine Dicke aufweist, die größer oder gleich der Dicke eines der Mehrzahl von Filamenten ist, und wobei die Dicke kleiner oder gleich der Dicke von zwei der Mehrzahl von Filamenten ist, wobei wenigstens einige der Mehrzahl von Kreuzungspunkten zwei der Mehrzahl von spiralförmigen Filamenten (702 und 704) umfassen, die miteinander verschmolzen sind, um eine elastische Verbindung (706) auszubilden, wobei die elastische Verbindung (706) dazu konfiguriert ist, sich zu biegen, wenn sich der röhrenförmige Körper zwischen der ausgedehnten Konfiguration und der zusammengefaltete Konfiguration bewegt, und die verbundenen Filamente daran gehindert werden, relativ zueinander zu gleiten.

2. Vorrichtung nach Anspruch 1, wobei jeder Kreuzungspunkt nicht mehr als zwei der Mehrzahl von spiralförmigen Filamenten (702, 704) umfasst.

3. Vorrichtung nach Anspruch 1 oder 2, wobei die Mehrzahl von Filamenten (702, 704) in einer einzigen, in Umfangsrichtung ausgerichteten ebenen Schicht angeordnet sind.

4. Vorrichtung nach einem der Ansprüche 1 bis 3, wobei die zwei spiralförmigen Filamente (702, 704) an dem Kreuzungspunkt einen Kreuzungswinkel ausbilden und wobei sich der Kreuzungswinkel während der Bewegung zwischen der ausgefahrenen und der zusammengefalteten Konfiguration auf bis zu 90 Grad ändert.

5. Vorrichtung nach einem der Ansprüche 1 bis 4, wobei wenigstens einige der Mehrzahlen von Kreuzungspunkten zwei der Mehrzahlen von spiralförmigen Filamenten umfassen, die sich ohne eine dazwischenliegende Befestigung kreuzen.

6. Vorrichtung nach einem der Ansprüche 1 bis 5, die ferner ein von dem röhrenförmigen Körper getragenes Therapeutikum umfasst, wobei das Therapeutikum dazu konfiguriert ist, von dort aus an das Gewebe an der Behandlungsstelle abgegeben zu werden.

7. Vorrichtung nach einem der Ansprüche 1 bis 6, die ferner einen Griff (1108) umfasst, der mit einem proximalen Ende des Innenschafts (1212) und einem proximalen Ende des Außenschafts (1216) gekoppelt ist, wobei der Griff (1108) ferner ein Aktuatorelement (1204) umfasst, wobei die Betätigung des Aktuatorelements (1204) den Innenschaft (1212) relativ zu dem Außenschaft (1216) bewegt, um den röhrenförmigen Körper auszudehnen oder zusammenfallen zu lassen.

8. Vorrichtung nach einem der Ansprüche 1 bis 7, wobei das erste Ende des rohrförmigen Körpers asymmetrisch mit dem Innenschaft (1212) gekoppelt ist oder das zweite Ende des rohrförmigen Schafts asymmetrisch mit dem Außenschaft (1216) gekoppelt ist.

9. Vorrichtung nach einem der Ansprüche 1 bis 8, wobei der röhrenförmige Körper eine proximale Öffnung (1506) und eine distale Öffnung (1508) umfasst und wobei die proximale und die distale Öffnung (1506 und 1508) in unterschiedlichen Ebenen liegen.

10. Vorrichtung nach einem der Ansprüche 1 bis 9, wobei ein Fluid oder ein Gas durch den rohrförmigen Körper ohne Einschränkung passiert oder diesen umfließt, wenn sich der rohrförmige Körper in der ausgedehnten Konfiguration oder in der zusammengefalteten Konfiguration befindet.

11. Vorrichtung nach einem der Ansprüche 1 bis 10, wobei der röhrenförmige Körper in der ausgedehnten Konfiguration eine konstante Kraft auf das Gewebe ausübt.

12. Vorrichtung nach einem der Ansprüche 1 bis 11, wobei der röhrenförmige Körper eine erste ausgedehnte Konfiguration und eine zweite ausgedehnte Konfiguration aufweist, und wobei in der ersten ausgedehnten Konfiguration eine Kraft auf das Gewebe ausgeübt wird und wobei in der zweiten ausgedehnten Konfiguration eine zweite Kraft, die größer ist als die erste Kraft, auf das Gewebe ausgeübt wird.

## Revendications

1. Dispositif de support de tissu temporaire et expansible, le dispositif comprenant :
une pluralité de filaments hélicoïdaux (702, 704) superposés les uns sur les autres pour former un corps tubulaire présentant une première extrémité, une seconde extrémité opposée à la première extrémité, et une lumière s'étendant entre la première extrémité et la seconde extrémité ;
une tige interne (1212) ; et
une tige externe (1216) disposée de manière glissante sur la tige interne (1212),
dans lequel la première extrémité est accouplée à la tige interne (1212), et la seconde extrémité est accouplée à la tige externe (1216),
dans lequel l'actionnement des tiges interne et externe (1212 et 1216) dans une première direction comprime la pluralité de filaments (702, 704), ce qui permet de provoquer une expansion radiale du corps tubulaire dans une configuration déployée qui est adaptée pour venir en contact avec et supporter un tissu au niveau d'un site de traitement sans obstruer l'écoulement d'un fluide au-delà du corps tubulaire, et
dans lequel l'actionnement des tiges interne et externe (1212 et 1216) dans une seconde direction opposée à la première direction met en tension la pluralité de filaments (702, 704), ce qui permet de replier radialement le corps tubulaire dans une configuration repliée qui est adaptée pour être mise en place sur le site de traitement ou retirée de celui-ci,
dans lequel la pluralité de filaments hélicoïdaux (702, 704) ont chacun une épaisseur et se croisent les uns les autres en une pluralité de points de croisement, et **caractérisé en ce que** chaque point de croisement présente une épaisseur supérieure ou égale à l'épaisseur d'un filament parmi la pluralité de filaments, et dans lequel l'épaisseur est inférieure ou égale à l'épaisseur de deux filaments parmi la pluralité de filaments, dans lequel au moins certains points de croisement de la pluralité de points de croisement comprennent deux filaments parmi la pluralité de filaments hélicoïdaux (702 et 704) fixés par fusion pour former un joint élastique (706), dans lequel le joint élastique (706) est conçu pour fléchir lorsque le corps tubulaire se déplace entre la configuration déployée et la configuration repliée et les filaments reliés sont empêchés de glisser l'un par rapport à l'autre.

2. Dispositif selon la revendication 1, dans lequel chaque point de croisement comprend au plus deux filaments parmi la pluralité de filaments hélicoïdaux (702, 704).

3. Dispositif selon la revendication 1 ou 2, dans lequel la pluralité de filaments (702, 704) est disposée dans une seule couche plane orientée circonférentiellement.

4. Dispositif selon l'une quelconque des revendications 1 à 3, dans lequel les deux filaments hélicoïdaux (702, 704) forment un angle de croisement au point de croisement, et dans lequel l'angle de croisement change de jusqu'à 90 degrés pendant le déplacement entre la configuration déployée et la configuration repliée.

5. Dispositif selon l'une quelconque des revendications 1 à 4, dans lequel au moins certains points de croisement parmi la pluralité de points de croisement comprennent deux filaments parmi la pluralité de filaments hélicoïdaux se croisant les uns les autres sans fixation entre eux.

6. Dispositif selon l'une quelconque des revendications 1 à 5, comprenant en outre un agent thérapeutique transporté par le corps tubulaire, dans lequel l'agent thérapeutique est conçu pour être distribué à partir de celui-ci au tissu au niveau du site de traitement.

7. Dispositif selon l'une quelconque des revendications 1 à 6, comprenant en outre une poignée (1108) accouplée à une extrémité proximale de la tige interne (1212) et une extrémité proximale de la tige externe (1216), la poignée (1108) comprenant en outre un élément actionneur (1204), dans lequel l'actionnement de l'élément actionneur (1204) déplace la tige interne (1212) par rapport à la tige externe (1216) pour provoquer l'expansion ou le repliement du corps tubulaire.

8. Dispositif selon l'une quelconque des revendications 1 à 7, dans lequel la première extrémité du corps tubulaire est accouplée à la tige interne (1212) de manière asymétrique ou la seconde extrémité de la tige tubulaire est accouplée à la tige externe (1216) de manière asymétrique.

9. Dispositif selon l'une quelconque des revendications 1 à 8, dans lequel le corps tubulaire comprend une ouverture proximale (1506) et une ouverture distale (1508), et dans lequel les ouvertures proximale et distale (1506 et 1508) sont situées dans des plans différents.

10. Dispositif selon l'une quelconque des revendications 1 à 9, dans lequel un fluide ou un gaz passe à travers le corps tubulaire ou autour de celui-ci sans restriction lorsque le corps tubulaire est dans la configuration déployée ou la configuration repliée.

11. Dispositif selon l'une quelconque des revendications 1 à 10, dans lequel le corps tubulaire dans la configuration déployée applique une force constante au tissu.

12. Dispositif selon l'une quelconque des revendications 1 à 11, dans lequel le corps tubulaire présente une première configuration déployée et une seconde configuration déployée, et dans lequel la première configuration déployée applique une force au tissu, et dans lequel la seconde configuration déployée applique au tissu une seconde force supérieure à la première force.
